# EUROPEAN PATENT APPLICATION

(11) **EP 1 683 519 A1**
(43) Date of publication of application: **26.07.2006**
(21) Application number: 04818519.3
(22) Date of filing: 12.11.2004
(51) Int. Cl.: A61K 31/202, A61K 31/232, A61P 25/28, A23L 1/30

(54) **PREVENTIVE/THERAPEUTIC AGENT FOR SPEECH DISORDER**

(30) Priority: 14.11.2003 JP 2003385129
(71) Applicant: MOCHIDA PHARMACEUTICAL CO., LTD., Shinjuku-ku Tokyo 160-8515 (JP)
(72) Inventor: OOTANI, Hiroaki, Etajima-shi, Hiroshima 7372211 (JP)
(74) Representative: Grünecker, Kinkeldey, Stockmair & Schwanhäusser Anwaltssozietät
(86) International application number: PCT/JP2004/016856
(87) International publication number: WO 2005/046668

(57) **Abstract**

A highly effective, safe agent for preventing and treating language disorders containing at least one member selected from the group consisting of icosapentaenoic acid and its salt and ester as an effective component is provided. The agent exhibits remarkable therapeutic effects for language disorder with no adverse side effects.

## Description

### TECHNICAL FIELD

This invention relates to a novel agent for preventing and treating language disorders, and more specifically, an agent for preventing and treating language disorders containing at least one member selected from the group consisting of icosapentaenoic acid (hereinafter referred to as EPA) and its salt and ester as an effective component. In the present invention, EPA is all-cis-5, 8, 11, 14, 17-icosapentaenoic acid.

### BACKGROUND ART

Language disorder in the broad sense is disorder of treating symbol information in human communication, and includes all disorders in the written and spoken languages. In clinical practice, language disorder in the narrow sense (for example, aphasia) means disorder of communication of content, and is distinguished from disorder of speech sounds in the spoken language, namely, speech disorder.
The method for treating the language disorder is different between the language disorder in the narrow sense and the speech disorder. However, both employ the basic approach of rehabilitation training, and no effective therapeutic agent has so far been reported for neither of such disorders.
EPA is a polyunsaturated fatty acid extracted and purified from fish oil, which is known to have the actions of reducing serum lipid level and suppressing platelet aggregation. In Japan, EPA is commercially purchased as a therapeutic agent for arteriosclerosis obliterans and hyperlipidemia.
EPA has been reported to have an action of enhancing production of nerve growth factor (NGF) (see, for example, Patent Document 1). Document 1 discloses that EPA has an action of enhancing NGF production in a cell derived from mouse connective tissue. Document 1, however, does not teach or indicate effectiveness of the EPA to the disorder of "language" which is one of the higher function of a human.

EPA has also been reported to have an action of enhancing brain function, learning ability, and memory, as well as ability to prevent and treat dementia (see, for example, Patent Document 2). Document 2 discloses that administration of EPA resulted in the increase of correct reaction rate in delayed discrimination test in rat. Document 2, however, also does not teach or indicate effectiveness of the EPA to the disorder of "language" which is one of the higher function of a human.
Furthermore, numerous reports other than those above have been published that disclose pharmacological action of the EPA. However, improvement of the language disorder by EPA is neither disclosed nor indicated in such publications.

Patent Document 1 : JP 8-143454 A
Patent Document 2 : JP 3-58926 A

### DISCLOSURE OF THE INVENTION

### PROBLEMS TO BE SOLVED BY THE INVENTION

In view of the situation as described above, an object of the present invention is to provide a highly safe agent for preventing and treating language disorders with higher therapeutic effects which can replace or which can be used simultaneously with the current treatment of the language disorder.

### MEANS TO SOLVE THE PROBLEMS

The inventors of the present invention conducted an extensive study on drugs that can be used for preventing and treating language disorders, and unexpectedly found a fact that EPA has therapeutic and prophylactic effects on language disorders with no adverse events. The present invention has been completed on the bases of such finding.

### EFFECT OF THE INVENTION

The agent for preventing and treating language disorders of the present invention containing EPA as its effective component exhibits remarkable therapeutic effects for language disorder with no adverse side effects, and therefore, it can be used as a highly effective, safe agent for preventing and treating language disorders.

### BEST MODE FOR CARRYING OUT THE INVENTION

Next, the present invention is described in detail.
An aspect of the present invention is directed to an agent for preventing and treating language disorders containing at least one member selected from the group consisting of icosapentaenoic acid and its salt and ester as an effective component. As long as therapeutic or prophylactic effect of the language disorders is obtained, ratio of the EPA in the total content of the fatty acids or form of the EPA is not particularly limited. However, ratio of the EPA in the total content of the fatty acids is typically at least 80% by mass, preferably at least 90% by mass, more preferably at least 95% by mass, and still more preferably at least 97% by mass. EPA is preferably in the form of EPA, ethyl ester of EPA (hereinafter referred to as EPA-E), sodium salt of EPA (hereinafter referred to as EPA-Na) or EPA glyceride (hereinafter referred to as EPA-G), and most preferably, in the form of EPA-E.
Another aspect of the present invention is directed to a food for preventing and treating language disorders containing at least one member selected from the group consisting of icosapentaenoic acid and its salt and ester as an effective component.

In the present invention, the term "language disorders" is used in the broad sense which include both the language disorders in the narrow sense and speech disorder. The agent for preventing and treating language disorders of the present invention is not limited for the underlying disease or the causative disease of the language disorders, or the disease associated with the language disorders. The language disorder associated with dementia is one exemplary therapeutic objective to which the agent for preventing and treating language disorders of the present invention is applicable. Of the various known types of dementia such as vascular-type dementia, dementia of the Alzheimer type, and mixed dementia, the language disorder associated with vascular-type dementia (for example, dementia developed after cerebral · infarction) is one exemplary preferable therapeutic objective to which the agent for preventing and treating language disorders of the present invention is applicable.

The EPA or its salt or ester used in the present invention is available as a commercial product having a EPA purity of 99% by mass or higher, a EPA-Na purity of about 99% by mass or higher, or a EPA-E purity of 98% by mass or higher. EPA may also be produced by purification from a fish oil or an EPA-producing bacterium or its culture medium by any of the methods known in the art such as continuous distillation, urea addition, liquid chromatography, supercritical fluid chromatography, and a combination thereof. If desired, the purified EPA may be esterified to produce a pharmaceutically acceptable ester such as an alkyl ester, for example, ethyl ester or a glyceride. EPA may also be produced in the form of a pharmaceutically acceptable salt with an inorganic base (for example, sodium salt or potassium salt); with an organic base (for example, benzylamine salt or diethylamine salt); or with a basic amino acid (for example, arginine salt or lysine salt). In the present invention, EPA includes EPA in the form of free fatty acid as well as the pharmaceutically acceptable salts and esters as described above unless otherwise noted.

When the agent for preventing and treating language disorders of the present invention contains a mixture of fatty acids other than the essential component (fatty acids other than EPA), content of the EPA in total fatty acid content is preferably high, and the EPA content in total fatty acid content is typically at least 80% by mass, preferably at least 90% by mass, more preferably at least 95% by mass, and still more preferably at least 97% by mass. Content of other long chain fatty acids is preferably low, and in particular, content of arachidonic acid is preferably as low as less than 3% by mass, more preferably less than 1% by mass, and still more preferably less than 0.5% by mass.

Exemplary fatty acids other than EPA include unsaturated fatty acids such as docosahexaenoic acid, docosapentaenoic acid, docosamonoenoic acid, arachidonic acid, eicosatetraenoic acid, eicosatrienoic acid, eicosamonoenoic acid, ocatadecatetraenoic acid, α-linolenic acid, linoleic acid, oleic acid, palmitoleic acid, hexadecatetraenoic acid, hexadecatrienoic acid and hexadecadienoic acid; and saturated fatty acids such as behenic acid, arachidic acid, stearic acid, palmitic acid, and myristic acid. The fatty acid as mentioned above may be in the form of free fatty acid as well as in the form of a salt with an inorganic base such as sodium salt, a salt with an organic base such as benzylamine salt or in the form of an ester, for example, an alkyl ester such as ethyl ester, or glyceride.

The effective component is preferably EPA-E, EPA-Na, or EPA-G. When the agent is orally administered, the effective component is preferably EPA-E, and when the agent is in an injection form, the effective component is preferably EPA-Na or EPA-G, and more preferably EPA-Na. Exemplary EPA-G include triicosapentaenoyl glyceride (hereinafter referred to as EPA-TG), 1,2-di(icosapentaenoyl) glyceride, 1,3-di(icosapentaenoyl) glyceride, 1-icosapentaenoyl glyceride, 2-icosapentaenoyl glyceride, a mixed glyceride wherein hydroxy group other than EPA group has been substituted with a medium chain fatty acid group other than EPA group, and mixtures thereof. Among these, the preferred are EPA-TG, diicosapentaenoyl glyceride, and diicosapentaenoyl mixed glyceride, and the more preferred is EPA-TG. The proportion of the EPA group in the total content of the long chain fatty acid residue in the glyceride is typically at least 80% by mass, preferably at least 90% by mass, more preferably at least 95% by mass, and most preferably 97% by mass.

The agent for preventing and treating language disorders of the present invention may be used by solely administering a compound of the effective component (and other possible components inevitably included in the process of purification), or in the form of an adequate medical preparation produced by combining the effective component with a carrier or medium commonly used in the art such as excipient, binder, lubricant, colorant, or flavoring agent, and optionally with sterilized water or vegetable oil, or further with a non-toxic organic solvent or non-toxic solubilizing agent (for example, glycerin or propylene glycol), emulsifier, suspending agent (for example, Tween 80 or gum arabic solution), isotonic agent, pH adjusting agent, stabilizer, or soothing agent.

Since EPA is highly unsaturated, the preparation as described above preferably further comprises an effective amount of an antioxidant such as butylated hydroxytoluene, butylated hydroxyanisole, propyl gallate, gallic acid, and pharmacologically acceptable quinone, and α-tocopherol.

The agent may be administered, for example, orally, intravenously, intraarterially, by inhalation, intrarectally, intravaginally, or externally to the patient and exemplary dosage forms include tablet, capsule, microcapsule, granules, fine granules, powder, oral liquid preparation, suppository, syrup, inhalant, ointment, injection (emulsion, suspension, or non-aqueous), and solid injection which is emulsified or suspended immediately before use. Preferably, the agent is orally administration after filling the agent in a capsule such as soft capsule or microcapsule. Also preferred are intravenous or intraarterial administration of an injection (emulsion, suspension, or non-aqueous), or a solid injection which is emulsified or suspended immediately before use. High purity EPA-E soft capsule and microcapsule (Epadel and Epadel S, both manufactured by Mochida Pharmaceutical Co., Ltd.) are already commercially purchased in japan as a safe therapeutic agent for arteriosclerosis obliterans and hyperlipidemia with reduced side effects.

The agent for preventing and treating language disorders of the present invention may be administered in a dose sufficient for developing the desired effect, and such dose may vary by the dosage form, administration route, frequency of doses a day, seriousness of the symptom, body weight, age, and the like. When orally administered, the dose in term of EPA is 300 to 9,000 mg/day, preferably 600 to 6,000 mg/day, and more preferably 1,800 to 2,700 mg/day, which is typically administered in 3 divided doses, but if desired, in a single dose or in several divided doses. When intravenously or intraarterially administered, the dose in terms of EPA is 1 to 200 mg, preferably 5 to 100 mg, and more preferably 10 to 50 mg, which is typically administered in single dose or in several divided doses, but if desired, in continuous manner for several hours to several days by infusion or by using an infusion pump.

The food for preventing and treating language disorders of the present invention is a food containing at least one member selected from the group consisting of icosapentaenoic acid and its salt and ester as an effective component. Non-limiting exemplary foods applied to the present invention include foods of animal origin, foods of vegetable origin, various processed foods, seasonings, beverages, and luxury items such as alcohols, and also, nutraceuticals such as supplements.
The food for preventing and treating language disorders of the present invention contains EPA or the like which is effective in treating the language disorders, and facilitates convenient ingestion of the EPA. If ingestion of the EPA is preliminarily enabled for those who has not yet developed the language disorders but are at high risk of developing the language disorders by such factor as aging and genetic factors, onset of the language disorders may be delayed, and prevention of the language disorders is thereby enabled.

### EXAMPLE

Next, the present invention is described in further detail by referring to the Example, which by no means limit the scope of the present invention.

### Example 1: Effect of EPA-E on a language-disordered patient with a history of cerebral infarction [Subject, history, and therapeutic history before EPA-E administration]

An 85 year old woman with the history of cerebral infarction was treated. The patient experienced cerebral infarction in November, 2001, and started rehabilitation therapy of left hemiplegia from April, 2002. In June, 2002, the patient was hospitalized for 1 month because of recurrence of the cerebral infarction, and again started the rehabilitation therapy from August, 2002. The drugs that the patient had been taking before the EPA-E include: Gramalil (trade name; manufactured by Fujisawa Pharmaceutical Co., Ltd.; generic name, tiapride hydrochloride), Ketas (trade name; manufactured by Kyorin Pharmaceutical Co., Ltd.; generic name, ibudilast), Panaldine (trade name; manufactured by Daiichi Pharmaceutical Co., Ltd.; generic name, ticlopidine hydrochloride), Nivadil (trade name; manufactured by Fujisawa Pharmaceutical Co., Ltd.; generic name, nilvadipine), Altat (trade name; manufactured by Teikoku Hormone Mfg. Co., Ltd.; generic name, roxatidine acetate hydrochloride).

### [Treatment with EPA-E and prognosis]

Before taking EPA-E, the patient could barely bring out a word. From September, 2002, the patient was administered with Epadel S600 (trade name; manufactured by Mochida Pharmaceutical Co., Ltd.; generic name, ethyl icosapentate) at a dose 600 mg and 3 times a day (namely, at a daily dose of 1800 mg).
From late October, 2002, the patient started to respond by saying "Good morning", and also started brief conversation with the care giver during the meal.
In November, 2002, the behavior of the patient like responding "not hurting anywhere" when fallen from the bed, and waking up at night and responding "I am not sleepy" were noted.
In December, 2002, the patient was able to carry on a complicated conversation comprising not only a single word but a plurality of words like talking with others "I am from ***. How about you". Frequency of the conversation also increased with the increase in the pronunciation clarity. The patient also responded to the care giver by saying "Thank you" and clearly asked "May I have some more (rice)?" in the meal.
In January, 2003, the frequency of the conversation as well as vocabulary increased even further.
In February, 2003, further improvement was noted in the frequency of conversation, vocabulary, communication ability, and the patient was discharged from the hospital in March, 2003. Since then, the patient was under home care service.

In the 6 months from the start of the administration, no adverse event suggested to be caused by EPA-E was found.
As described above, EPA exhibited significant therapeutic effects for language disorder with no side effects, indicating that is a highly effective, safe agent for preventing and treating language disorders.

## Claims

1. An agent for preventing and treating language disorders containing at least one member selected from the group consisting of icosapentaenoic acid and its salt and ester as an effective component.

2. The agent for preventing and treating language disorders according to claim 1 wherein the effective component is included as a component of a fatty acid mixture, and content of icosapentaenoic acid and its salt and ester in total content of the fatty acid is not less than 80% by mass.

3. The agent for preventing and treating language disorders according to claim 1 or 2 wherein the agent contains ethyl ester of the icosapentaenoic acid as its effective component.

4. The agent for preventing and treating language disorders according to any one of claims 1 to 3 wherein daily dose of the effective component is in the range of 1,800 to 2,700 mg.

5. The agent for preventing and treating language disorders according to any one of claims 1 to 4 wherein the language disorders is the one associated with dementia.

6. A food for preventing and treating language disorders containing at least one member selected from the group consisting of icosapentaenoic acid and its salt and ester as an effective component.
